# EUROPEAN PATENT SPECIFICATION

(11) **EP 4 304 491 B1**
(45) Date of publication and mention of the grant of the patent: **18.11.2026**
(21) Application number: 22728702.6
(22) Date of filing: 03.05.2022
(51) Int. Cl.: A61B 17/08, A61B 17/02, A61B 17/10

(54) **REPOSITIONABLE TISSUE-ENGAGEMENT MEMBER AND ASSOCIATED SYSTEMS**
REPOSITIONIERBARES GEWEBEEINRASTELEMENT SOWIE ZUGEHÖRIGE SYSTEME
ÉLÉMENT DE MISE EN PRISE DE TISSU REPOSITIONNABLE ET SYSTÈMES

(30) Priority: 04.05.2021 US 202163183790 P
(43) Date of publication of application: 17.01.2024
(73) Proprietor: Boston Scientific Scimed, Inc., Maple Grove, MN 55311 (US); Boston Scientific Medical Device Limited, Galway (IE)
(72) Inventor: BAGLEY, Kevin L., Natick, Massachusetts 01760 (US); SHARMA, Deepak Kumar, Muzaffarnagar 251001 (IN); RAUT, Shrikant Vasant, Mumbai 401204 (IN); SCUTTI, James J., Norwell, Massachusetts 02061 (US); MISHRA, Agrim, New Delhi 110059 (IN); BHOWMICK, Nabarun, Kolkata 700108 (IN)
(74) Representative: Vossius & Partner Patentanwälte Rechtsanwälte mbB
(86) International application number: PCT/US2022/027444
(87) International publication number: WO 2022/235649

(56) References cited:
- WO-A1-2021/000782
- US-A1- 2019 059 905

## Description

### FIELD

The present disclosure relates generally to the field of tissue-engagement members and associated systems and methods. More particularly, the present disclosure relates to the field of repositionable tissue-engagement members and associated systems and methods.

### BACKGROUND

Physicians are becoming more proficient at endoscopic procedures using transluminally inserted tools to perform various procedures without the need for open surgery. However, with currently available technologies, conducting more complex procedures, such as endoscopic submucosal dissection (ESD) or an endoscopic mucosal resection (EMR) (which may be used to remove lesions from the gastrointestinal tract), can be difficult and time consuming. Manipulating devices for clipping tissue may require procedurally complicated and/or time-intensive techniques for positioning and orienting and re-orienting the devices and associated medical instruments. In addition, non-ideal visualization and lack of tissue tension also make the procedures difficult and time consuming. Having a means to create more significant traction force on the tissue would be desirable to improve the visualization of the cutting plane. The use of repositionable clips has become important for improving the ability to re-tension a tether losing tension as a procedure (e.g., cutting) on grasped tissue is performed. Also, repositionable clips allow for adjustment during a procedure, and facilitate device retrieval. However, current repositionable clips present challenges with respect to such factors as cost, usability, and required cognitive load for manipulation / actuation thereof.
WO 2021/000782 A1 describes a medical device capable of repeatedly opening and closing, comprising: a closing clip; a clip base; a control wire; an outer sleeve; an actuator; and a releasable connecting member. The releasable connecting member has a largely tubular structure, and has a radially expandable structure on a distal end side thereof. A distal end part of the expandable structure is engaged with an outer wall of a proximal end part of the clip base. A distal end of the control wire is provided with an expansion actuation component, and when the control wire is pulled toward a proximal end, the expansion actuating component abuts against the expandable structure and drives the distal end side of the expandable structure to expand radially, thereby releasing the clip base.
US 2019/059905 A1 describes a medical device used to cause hemostasis of blood vessels using a clip arrangement delivered to a target region through an endoscope. The medical device includes a reversibly closeable clip, a locking arrangement, a control wire, a sheath, and a handle with an actuating trigger.

It is with the above considerations in mind that the improvements of the present disclosure may be useful.

### SUMMARY

This summary of the disclosure is given to aid understanding, and one of skill in the art will understand that each of the various aspects and features of the disclosure may advantageously be used separately in some instances, or in combination with other aspects and features of the disclosure in other instances. No limitation as to the scope of the claimed subject matter is intended by either the inclusion or non-inclusion of elements, components, or the like in this summary.

In accordance with various principles of the present disclosure, a tissue-engagement member includes a pair of jaw members coupled together and movable between a closed configuration and an open configuration. The pair of jaw members also includes an actuator operatively engaged with the pair of jaw members to shift the jaw members between the closed configuration and the open configuration. The pair of jaw members also includes a biasing element positioned to cause the jaw members to be biased into the closed configuration. The pair of jaw members also includes a cam mechanism operatively associated with at least one of the actuator, the biasing element, or at least one of the jaw members.

In some embodiments, the actuator is shiftable against the force of the biasing element to shift the jaw members from the closed configuration to the open configuration. In some embodiments, the biasing element operatively engages at least one of the actuator or at least one of the jaw members. In some embodiments, the biasing element is positioned about a portion of one of the actuator or at least one of the jaw members.

In some embodiments, the cam mechanism includes a cam surface on one of the actuator or at least one of the jaw members. In some embodiments, the cam mechanism further includes a cam follower on the other of the actuator or at least one of the jaw members. In some embodiments, the biasing element operatively engages one of the actuator or at least one of the jaw members to actuate the cam mechanism to return the jaw members to a closed configuration from an open configuration.

In some embodiments, the tissue-engagement member further includes a housing, the jaw member and the actuator positioned within a portion of the housing. In some embodiments, the biasing element operatively engages one of the actuator or the housing or at least one of the jaw members to bias the jaw members to a closed configuration.

In some embodiments, the jaw members are coupled together and biased apart from each other, the actuator is movable between a position holding the jaw members in the closed configuration and a position allowing the jaw members to move apart to the open configuration, and the biasing element biases the actuator into the position holding the jaw members in the closed configuration.

In some embodiments, the tissue-engagement member further includes an actuation element coupled to the actuator and graspable to move the actuation element to shift the jaw members between the closed and open configurations.

In accordance with various principles of the present disclosure, a tissue-engagement member includes a pair of jaw members coupled together and movable between a closed configuration and an open configuration, an actuator operatively engaged with the pair of jaw members to shift the jaw members between the closed configuration and the open configuration, and a biasing element positioned over a portion of at least one of the actuator or the jaw members to cause the jaw members to be biased into the closed configuration.

In some embodiments, the biasing element is an elastic element positioned about the jaw members.

In some embodiments, the biasing element is positioned about a portion of the actuator.

In some embodiments, the tissue-engagement member further includes a cam mechanism operatively associated with at least one of the actuator, the biasing element, or at least one of the jaw members. In some embodiments, the cam mechanism includes a cam surface on one of the actuator or at least one of the jaw members. In some embodiments, the cam mechanism further includes a cam follower on the other of the actuator or at least one of the jaw members.

In accordance with various principles of the present disclosure a tissue-engagement system includes a tissue-engagement member having a distal end and a proximal end, the tissue-engagement member including jaws along the distal end, an actuator along the proximal end, and a biasing element, where the jaws are shiftable between a closed configuration and an open configuration, the actuator is operatively engaged with the jaws to move the jaws between the closed and open configurations, and the biasing element biases the jaws into the closed configuration. The tissue-engagement system further includes a tissue-engagement-member manipulator having a distal end configured to operatively engage with the tissue-engagement member to move the actuator to shift the jaws between the closed and open configurations, and a manipulator deployment system configured to deliver and to maneuver the tissue-engagement-member manipulator to move the actuator.

In some embodiments, an actuation element is operatively coupled with a proximal end of the actuator along the proximal end of the tissue-engagement member; and an actuator-engagement element is provided on a distal end of the tissue-engagement-member manipulator and configured to engage with the actuation element in a range of orientations with respect to the actuation element.

In some embodiments, the manipulator deployment system further includes a flexible tubular element with a lumen defined therethrough, and a sheath, where the tissue-engagement-member manipulator is movable proximally into the lumen defined through the flexible tubular element to bring the proximal end of the tissue-engagement member into abutment with a distal end of the flexible tubular element, and the sheath is movable over the distal end of the flexible tubular element and over the tissue-engagement member to hold the tissue-engagement member in alignment with the manipulator deployment system.

These and other features and advantages of the present disclosure, will be readily apparent from the following detailed description, the scope of the claimed invention being set out in the appended claims. While the following disclosure is presented in terms of aspects or embodiments, it should be appreciated that individual aspects can be claimed separately or in combination with aspects and features of that embodiment or any other embodiment.

### BRIEF DESCRIPTION OF THE DRAWINGS

Non-limiting embodiments of the present disclosure are described by way of example with reference to the accompanying drawings, which are schematic and not intended to be drawn to scale. The accompanying drawings are provided for purposes of illustration only, and the dimensions, positions, order, and relative sizes reflected in the figures in the drawings may vary. For example, devices may be enlarged so that detail is discernable, but is intended to be scaled down in relation to, *e.g.,* fit within a working channel of a delivery catheter or endoscope. In the figures, identical or nearly identical or equivalent elements are typically represented by the same reference characters, and similar elements are typically designated with similar reference numbers differing in increments of 100, with redundant description omitted. For purposes of clarity and simplicity, not every element is labeled in every figure, nor is every element of each embodiment shown where illustration is not necessary to allow those of ordinary skill in the art to understand the disclosure.

The detailed description will be better understood in conjunction with the accompanying drawings, wherein like reference characters represent like elements, as follows:
**FIG. 1** illustrates a perspective view of an example of an embodiment of a tissue-engagement member and associated system in accordance with various aspects of the present disclosure.
**FIG. 2** illustrates a perspective view of a tissue-engagement member as in **FIG. 1****.**
**FIG. 3** illustrates an elevational view of a tissue-engagement member as in **FIG. 2** with an outer portion in phantom to reveal internal components, and showing the tissue grasping portions in a closed configuration.
**FIG. 4** illustrates an elevational view of a tissue-engagement member as in **FIG. 2** and **FIG. 3** with an outer portion in phantom to reveal internal components, and showing the tissue grasping portions in an open configuration.
**FIG. 5** illustrates a perspective view of another embodiment of a tissue-engagement member which may be used with a system as illustrated in **FIG. 1****.**
**FIG. 6** illustrates an elevational view of a tissue-engagement member as in **FIG. 5** with an outer portion in phantom to reveal internal components, and showing the tissue grasping portions in a closed configuration.
**FIG. 7** illustrates an elevational view of a tissue-engagement member as in **FIG. 5** and **FIG. 6** with an outer portion in phantom to reveal internal components, and showing the tissue grasping portions in an open configuration.
**FIG. 8** illustrates a perspective view of another embodiment of a tissue-engagement member which may be used with a system as illustrated in **FIG. 1****.**
**FIG. 9** illustrates an elevational view of a tissue-engagement member as in **FIG. 8** with an outer portion in phantom to reveal internal components, and showing the tissue grasping portions in a closed configuration.
**FIG. 10** illustrates an elevational view of a tissue-engagement member as in **FIG. 8** and **FIG. 9** with an outer portion in phantom to reveal internal components, and showing the tissue grasping portions in an open configuration.
**FIG. 11** illustrates a perspective view of another embodiment of a tissue-engagement member which may be used with a system as illustrated in **FIG. 1****.**
**FIG. 12** illustrates an elevational view of a tissue-engagement member as in **FIG. 11** with an outer portion in phantom to reveal internal components, and showing the tissue grasping portions in a closed configuration.
**FIG. 13** illustrates a perspective view of a tissue-engagement member as in **FIG. 11** and **FIG. 12** with an outer portion in phantom to reveal internal components, and showing the tissue grasping portions in a closed configuration.
**FIG. 14** illustrates a perspective view of another embodiment of a tissue-engagement member which may be used with a system as illustrated in **FIG. 1****.**
**FIG. 15** illustrates a cross-sectional view along line **XV-XV** of **FIG. 14** of an embodiment of a tissue-engagement member as in **FIG. 14****.**
**FIG. 16** illustrates a perspective view of a tissue-engagement member as in **FIG. 14** and **FIG. 15** with an outer portion in phantom to reveal internal components, and showing the tissue grasping portions in a closed configuration.
**FIG. 17** illustrates a perspective view of a tissue-engagement member as in **FIGS. 14-****16** with an outer portion in phantom to reveal internal components, and showing the tissue grasping portions in an open configuration.
**FIG. 18** illustrates a perspective view of a variation of a tissue-engagement member as in **FIGS. 14-17** and which may be used with a system as illustrated in **FIG. 1****.**
**FIG. 19-21** illustrate sequential positions of a tissue-engagement member and a tissue-engagement-member manipulator from a closed configuration of the tissue-engagement member to an open configuration of the tissue-engagement member.

### DETAILED DESCRIPTION

The following detailed description should be read with reference to the drawings, which depict illustrative embodiments. It is to be understood that the disclosure is not limited to the particular embodiments described, as such may vary. All apparatuses and systems and methods discussed herein are examples of apparatuses and/or systems and/or methods implemented in accordance with one or more principles of this disclosure. Each example of an embodiment is provided by way of explanation and is not the only way to implement these principles but are merely examples. Thus, references to elements or structures or features in the drawings must be appreciated as references to examples of embodiments of the disclosure, and should not be understood as limiting the disclosure to the specific elements, structures, or features illustrated. Other examples of manners of implementing the disclosed principles will occur to a person of ordinary skill in the art upon reading this disclosure. In fact, it will be apparent to those skilled in the art that various modifications and variations can be made in the present disclosure without departing from the scope of the present subject matter. For instance, features illustrated or described as part of one embodiment can be used with another embodiment to yield a still further embodiment. Thus, it is intended that the present subject matter covers such modifications and variations as come within the scope of the appended claims.

It will be appreciated that the present disclosure is set forth in various levels of detail in this application. In certain instances, details that are not necessary for one of ordinary skill in the art to understand the disclosure, or that render other details difficult to perceive may have been omitted. The terminology used herein is for the purpose of describing particular embodiments only, and is not intended to be limiting beyond the scope of the appended claims. Unless defined otherwise, technical terms used herein are to be understood as commonly understood by one of ordinary skill in the art to which the disclosure belongs. All of the devices disclosed and claimed herein can be made and executed without undue experimentation in light of the present disclosure.

As used herein, "proximal" refers to the direction or location closest to the user (medical professional or clinician or technician or operator or physician, etc., such terms being used interchangeably herein without intent to limit, and including automated controller systems or otherwise), etc., such as when using a device (e.g., introducing the device into a patient, or during implantation, positioning, or delivery), and "distal" refers to the direction or location furthest from the user, such as when using the device (e.g., introducing the device into a patient, or during implantation, positioning, or delivery). "Longitudinal" means extending along the longer or larger dimension of an element. "Central" means at least generally bisecting a center point, and a "central axis" means, with respect to an opening, a line that at least generally bisects a center point of the opening, extending longitudinally along the length of the opening when the opening comprises, for example, a tubular element, a channel, a cavity, or a bore.

In accordance with various principles of the present disclosure, a tissue-engagement member has a first tissue-engaging element and a second tissue-engaging element movably coupled with respect to each other to engage, contact, grip, grasp, etc., with body tissue. For the sake of convenience, and without intent to limit, reference will be made to a jaw rather than a tissue-engaging element. The jaws of a tissue-engagement member formed in accordance with various principles of the present disclosure may include a pair of jaw members which are movable with respect to each other between a closed configuration, in which the jaw members are in contact with each other or are substantially in contact (e.g., with tissue grasped therebetween) with each other, and an open configuration in which the jaw member are spaced apart (e.g., to allow tissue to be positioned therebetween or to be removed or released from between the jaw members). It will be appreciated that a tissue-engagement member formed in accordance with various principles of the present disclosure may also be referenced as a clip, clasp, fastener, clamp, etc., without intent to limit. Reference may be made herein simply to the jaws, rather than the jaw members, for the sake of simplicity and without intent to limit.

In accordance with various principles of the present disclosure, a biasing element is provided to maintain the jaws of a tissue-engagement member in a closed configuration. In some embodiments, the jaws are in a closed configuration when the biasing element in a rest position. It will be appreciated that terms such as rest, neutral, unbiased, undeformed, etc., may be used interchangeably herein to indicate a state with no built up or stored potential energy. It will further be appreciated that terms such as configuration and position (and conjugations and other grammatical forms thereof) may be used interchangeably herein without intent to limit. The biasing element may be formed as a separate element (e.g., separately from the jaws of the tissue-engagement member). The biasing element may be in the form of an elastic band or a spring-biased metal element.

In embodiments in which the biasing element maintains the jaws in a closed configuration when the biasing element is in a rest position, the biasing element must be shifted into another configuration or position, such as a potential-energy-storing configuration, to allow the jaws to shift to an open configuration. In some embodiments, an actuator may be provided to move the jaws into an open configuration, such as by moving the biasing element into a potential-energy-storing configuration. In some embodiments, the actuator moves the jaws against the force of the biasing element. In some embodiments, the actuator moves against the force of the biasing element.

In some embodiments, the jaws of the tissue-engagement member are pivotably coupled together and a biasing element maintains the jaws in a closed configuration. An actuator may be provided in such embodiments to move the jaws against the biasing force of the biasing element into an open configuration and to hold jaws in the open configuration. When the actuator is released, the biasing element may be positioned and/or configured to return the jaws to the closed configuration.

In some embodiments, a cam mechanism is associated with at least one of the actuator, the biasing element, or at least one of the jaw members. The cam mechanism may include a cam surface and a cam follower. In some embodiments, a cam surface is provided on one of either the actuator or a jaw member (one or both of the jaw members), and a cam follower is provided on the other of the actuator or a jaw member (one or both of the jaw members). Movement of the actuator with respect to the jaw members causes the cam follower to move along the cam surface to cause the jaw members to move between the closed configuration and the open configuration.

In some embodiments, the jaws are biased into an open configuration, and the biasing element maintains the jaws in a closed configuration. For instance, the biasing element may bias an actuator into a jaw-closing position to hold the jaws of the tissue-engagement member in the closed configuration. In some embodiments, an actuator is positionable over the jaws to hold the jaws in a closed configuration. The actuator may be shifted to allow the jaws to shift to an open configuration, such as by being shifted against the biasing element. When the actuator is released, the biasing element may be positioned and/or configured to return the actuator back to the jaw-closing position to shift the jaws back to the closed configuration.

In some embodiments, the biasing element is provided over a portion of the tissue-engagement member. In some embodiments, the biasing element contacts an outer surface of the tissue-engagement member. In some embodiments, the biasing element contacts the jaws of the tissue-engagement member. In some embodiments, an actuator is operatively associated with the jaws to cause the jaws to move between the closed and open configurations, and the biasing element contacts the actuator. In some embodiments, the biasing element surrounds a portion of the tissue-engagement member. In some embodiments, the biasing element surrounds the jaws of the tissue-engagement member. In some embodiments, an actuator is operatively associated with the jaws to cause the jaws to move between the closed and open configurations, and the biasing element surrounds the actuator. In some embodiments, the biasing element contacts and/or surrounds at least one of the jaws or an actuator of the tissue-engagement member.

As noted above, in some embodiments an actuator is provided to shift the jaws between the closed configuration and the open configuration. The actuator may be coupled or linked with the jaws to move the jaws between the open and closed configurations, or may operatively engage the jaws without being linked with the jaws. The actuator may be positioned with respect to the jaws such that linear movement of the actuator causes the jaws to shift between a closed configuration and an open configuration.

An actuation element may be provided and arranged and configured to facilitate operative engagement of the actuator to cause the tissue-engagement member to shift between open and closed configurations. The actuation element may be an actuator-engagement element configured to facilitate operative engagement with the actuator. However, other configurations of actuation elements are within the scope of the present disclosure. Reference may be made herein to provided, associated, coupled, engaged, etc. to describe physical relationships of the actuation element with at least one part or component of the tissue-engagement member, such terms being used interchangeably herein without intent to limit unless otherwise indicated. The actuation element is configured to be operatively engaged by an instrument with an actuator-engaging element. It will be appreciated that terms such as associated, coupled, engaged, contacted, connected, linked, etc. (including conjugations and other grammatical forms thereof) may be used interchangeably herein without intent to limit unless otherwise indicated. In some embodiments, the actuation element is configured to facilitate operative engagement with an actuator-engaging element without regard to the orientation of the tissue-engagement member with respect to tissue being grasped by the jaws of the tissue-engagement member. In some embodiments, the actuator-engaging element may operatively engage the actuation element in more than one orientation with respect to the actuation element. In some embodiments, the actuation element is configured to facilitate operative engagement with the actuator-engaging element when the actuator-engaging element is positioned within a range of angular positions with respect to the actuation element. For instance, the actuation element may be configured to allow operative engagement with the actuator-engaging element when the actuator-engaging element is positioned up to about 90° from an aligned (e.g., ideally aligned) position with respect to the actuation element. In accordance with various principles of the present disclosure, a tissue-engagement-member manipulator is provided with an actuator-engaging element configured to operatively engage the actuation element.

In accordance with various principles of the present disclosure, one or more tissue-engagement members are provided in a tissue-engagement system. The system may also include a tissue-engagement-member manipulator configured to actuate at least one of the tissue-engagement members to shift the jaws of the tissue-engagement member between closed and open configurations. The tissue-engagement-member manipulator may be pre-loaded in operative engagement with a tissue-engagement member to facilitate quick deployment of the tissue-engagement member. The system may further include a deployment system configured to deliver and maneuver the a tissue-engagement-member manipulator to deploy and/or maneuver a tissue-engagement member.

Various embodiments of tissue-engagement members will now be described with reference to examples illustrated in the accompanying drawings. Reference in this specification to "one embodiment," "an embodiment," "some embodiments", "other embodiments", etc. indicates that one or more particular features, structures, and/or characteristics in accordance with principles of the present disclosure may be included in connection with the embodiment. However, such references do not necessarily mean that all embodiments include the particular features, structures, and/or characteristics, or that an embodiment includes all features, structures, and/or characteristics. Some embodiments may include one or more such features, structures, and/or characteristics, in various combinations thereof. Moreover, references to "one embodiment," "an embodiment," "some embodiments", "other embodiments", etc. in various places in the specification are not necessarily all referring to the same embodiment, nor are separate or alternative embodiments necessarily mutually exclusive of other embodiments. When particular features, structures, and/or characteristics are described in connection with one embodiment, it should be understood that such features, structures, and/or characteristics may also be used in connection with other embodiments whether or not explicitly described, unless clearly stated to the contrary. It should further be understood that such features, structures, and/or characteristics may be used or present singly or in various combinations with one another to create alternative embodiments which are considered part of the present disclosure, as it would be too cumbersome to describe all of the numerous possible combinations and subcombinations of features, structures, and/or characteristics. Moreover, various features, structures, and/or characteristics are described which may be exhibited by some embodiments and not by others. Similarly, various features, structures, and/or characteristics or requirements are described which may be features, structures, and/or characteristics or requirements for some embodiments but may not be features, structures, and/or characteristics or requirements for other embodiments. Therefore, the present invention is not limited to only the embodiments specifically described herein.

It will be appreciated that in the following description, elements or components similar among the various illustrated embodiments are generally designated with the same reference numbers increased by 100 and redundant description is omitted. Description of similar elements with similar descriptions may be omitted for the sake of brevity, reference being made to description of one element as applicable to another similar element. Common features are identified by common reference elements and, for the sake of brevity, the descriptions of the common features are generally not repeated. For purposes of clarity, not all components having the same reference number are numbered.

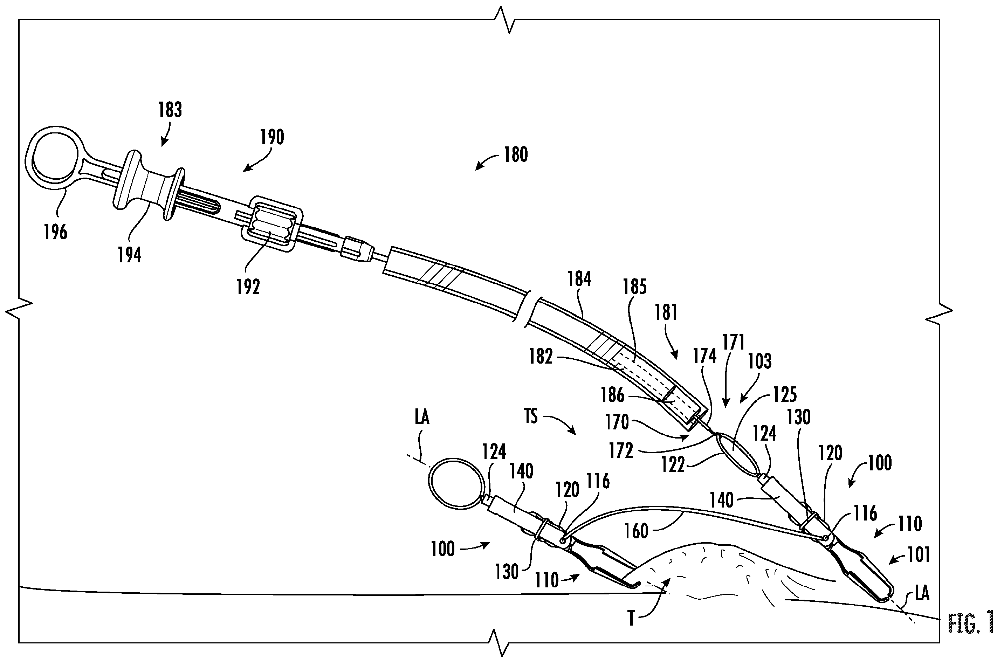

Turning now to the drawings, an example of an embodiment of a tissue-engagement member **100** formed in accordance with various principles of the present disclosure is illustrated grasping tissue **T.** Another example of an embodiment of a tissue-engagement member **100** formed in accordance with various principles of the present disclosure is illustrated in **FIG. 1** being operatively engaged by a tissue-engagement-member manipulator **170.** The tissue-engagement member **100** in the illustration of **FIG. 1** may be used to impart traction to tissue with the use of a tether **160** extending between (and joining) the tissue-engagement members **100** in a manner known or heretofore known. The tether **160** may be traction band, elastic band, stretchable elongate member, wire, cord, cable, spring, suture, and/or any other suitable elongate member, which optionally is stretchable or elongatable. It will be appreciated that other uses of a tissue-engagement member formed in accordance with various principles of the present disclosure are within the scope of the present disclosure. Moreover, it will be appreciated that although **FIG. 1** illustrates one of the embodiments of a tissue-engagement member **100** disclosed herein, any of the other tissue-engagement member **200, 300, 400, 500, 500'** may be illustrated in a similar environment for use and manipulation in a similar manner.

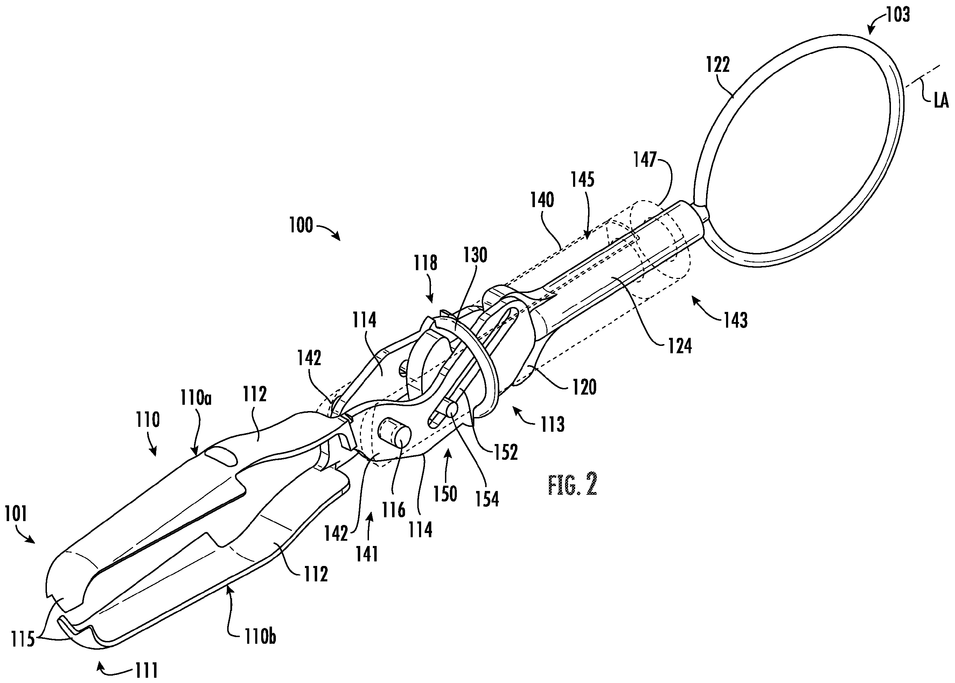

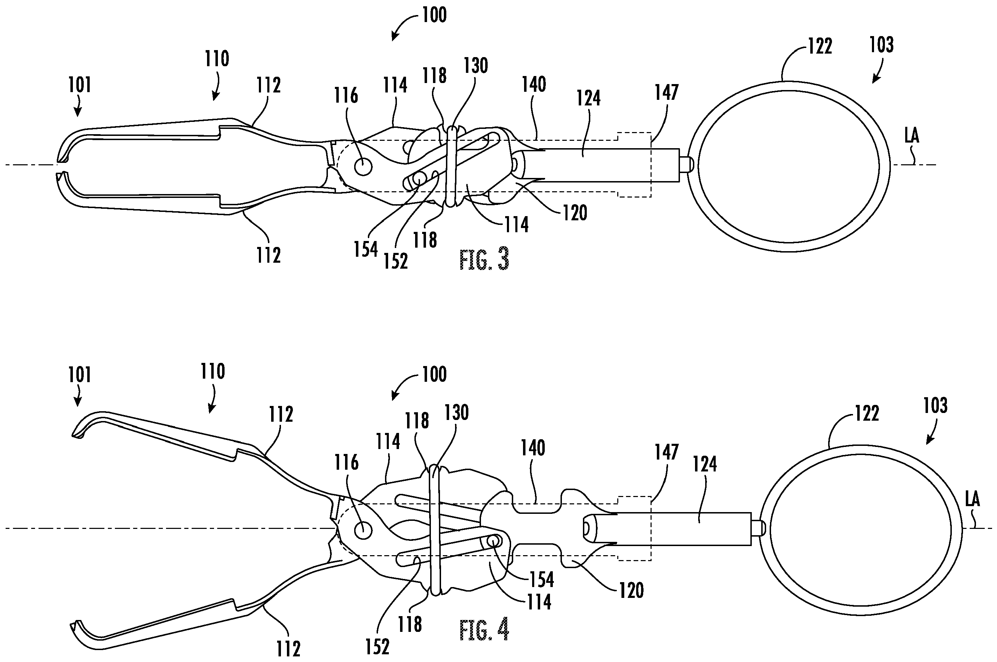

The tissue-engagement member **100** illustrated in **FIG. 1** has tissue-engaging jaws **110** along a distal end **101** of the tissue-engagement member **100.** The jaws **110** are moveable between a closed configuration (as shown in **FIG. 3****,** and as illustrated in **FIG. 1** with tissue **T** grasped between the jaws **110**) and an open configuration (as shown in **FIG. 4**). An actuator **120** (shown in greater detail in **FIGS. 2-4****)** is operatively associated with the jaws **110** to cause the jaws **110** to move between the open and closed configurations. In the illustrated embodiment, a biasing element **130** maintains the jaws **110** in the closed configuration. The biasing element **130** may be in the form of a rubber band, o-ring, or other elastic element capable of being positioned in operative association with, such as around, the jaws **110.** A casing or housing **140** (hereinafter housing, for the sake of convenience and without intent to limit), such as in the form of a clevis, may be positioned about the jaws **110** and actuator **120.** In the illustrated embodiment, the biasing element **130** extends about the housing **140.** The housing **140** may be formed by a relatively simple stamping process, facilitating and lowering the manufacturing cost of the tissue-engagement member **100.**

In accordance with various principles of the present disclosure, an actuation element **122** is operatively associated with a proximal end **103** of the tissue-engagement member **100** to facilitate actuation of the tissue-engagement member **100** to shift between the closed and open configurations. In the illustrated embodiment, the actuation element **122** is in the form of a loop which may readily be engaged by an actuator-engaging element **172** of a tissue-engagement-member manipulator **170,** as discussed in further detail below. However, other configurations of an actuation element **122** permitting ready engagement thereof are within the scope of the present disclosure. In accordance with various principles of the present disclosure, the actuation element **122** may be moved longitudinally along the longitudinal axis **LA** of the tissue-engagement member **100** to actuate the actuator **120** to shift the jaws **110** between the open and closed configurations. The manner in which the tissue-engagement member **100** operates and the mechanism by which the actuator **120** and jaws **110** interact in the example of an embodiment of a tissue-engagement member **100** illustrated in **FIG. 1** will be better appreciated with reference to **FIGS. 2-4****.**

A perspective view of an example of an embodiment of a tissue-engagement member **100** is illustrated in **FIG. 2** with its housing **140** in phantom to reveal the arrangement of elements of the tissue-engagement member **100** resulting in the desired operational characteristics of the tissue-engagement member **100.**

The jaws **110** of the tissue-engagement member **100** include a pair of jaw members **110a, 110b** each having a tissue-engaging end **112** (functionally, the ends between which an element, such as tissue, is to be grasped) along a distal end **111** of the jaws **110.** The tissue-engaging distal ends **111** of the jaws **110** are illustrated as having interengaging teeth **115,** although other configurations are within the scope of the present disclosure. A shank **114** (which may be referenced as a shaft or wing without intent to limit) extends proximally from each tissue-engaging end **112** along the proximal end **113** of the jaw members **110a, 110b.** As may be seen through the phantom depiction of the housing **140,** the shanks **114** of the jaws **110** extend between the prongs or legs or arms **142** (hereinafter arms, for the sake of convenience and without intent to limit) of the generally clevis-shaped housing **140.** The jaw members **110a, 110b** of the jaws **110** are pivotable about a pivot pin **116** to pivot with respect to each other between closed and open configurations. The pivot pin **116** may be mounted with respect to the distal end **141** of the housing **140** to maintain the jaws **110** mounted with respect to the housing **140.** It will be appreciated that the pivot pin **116** may extend through both jaw members **110a, 110b,** or each jaw member **110a, 110b** may have a respective outwardly extending pivot pin **116** facing towards and mounted with respect to an arm **142** of the housing **140.** It is noted that a tether **160** may be coupled to the tissue-engagement member **100** such as via the pivot pin **116,** as illustrated in the embodiment of **FIG. 1****,** and optionally via a hinge or other extension coupled to or formed with the pivot pin **116.** Other configurations, such as coupling via the housing **140,** are within the scope of the present disclosure.

The actuator **120** is operatively associated with the jaws **110** to effect shifting of the jaws **110** between the closed and open configurations as will be described in further detail below, such as with reference to **FIG. 3** and **FIG. 4****.** The actuator **120** is positioned adjacent the shanks **114** of the jaws **110** and between the arms **142** of the housing **140.** The actuator **120** may be larger in at least one dimension than the diameter of the housing **140** and thus may extend through a window **145** provided in the housing **140** to allow proximal sliding of the actuator **120** to actuate the jaws **110.**

A cam mechanism **150** is operatively associated with the actuator **120** and the jaws **110** of the tissue-engagement member **100.** As may be seen through the phantom lines of the housing **140,** the cam mechanism **150** of the example of an embodiment of a tissue-engagement member **100** illustrated in **FIG. 2** associates the actuator **120** with the proximal end **113** of the jaws **110** generally within the window **145** of the housing **140** between the housing arms **142.** The cam mechanism **150** is configured and arranged so that movement of the actuator **120** shifts the jaws **110** between the closed configuration illustrated in **FIG. 3** and the open configuration illustrated in **FIG. 4****.** More particularly, the cam mechanism **150** includes a cam surface **152** and a cam follower **154** which moves along cam surface **152.** It will be appreciated that various terms may be used interchangeably hereinto describe the motion of the cam follower **154,** such as ride, slide, extend, translate, shift, etc., without intent to limit. In the illustrated embodiment, the cam surface **152** is in the form of slots in the respective shanks **114** along the respective proximal ends **113** of the jaw members **110a, 110b,** and the cam follower **154** is in the form of a pin extending from either side of the actuator **120** facing a jaw member **110a, 110b** to slide within a respective slot. It will be appreciated that a reverse configuration (with inwardly-facing, actuator-facing pins on the shanks **114** and respective slots for the pins on outwardly-facing, shank-facing sides of the actuator **120**) or other configurations of cam mechanism **150** are within the scope of the present disclosure.

Operation of the cam mechanism **150** may be more readily appreciated with reference to **FIG. 3** and **FIG. 4****.** A biasing element **130** is operatively associated with the jaws **110** and interacts with the jaws **110** to hold the jaws **110** in a closed configuration with the cam mechanism **150** in a closed configuration, as illustrated in **FIG. 3****.** In the illustrated embodiment, the biasing element **130** is positioned about the shanks **114** of the jaws **110** along the proximal end **103** of the jaws **110** to encircle or surround the shanks **114,** and the biasing element **130** operatively engages the jaws **110.** However, other positions and configurations of a biasing element **130** are within the scope of the present disclosure. As described above, the tissue-engagement member **100** includes an actuation element **122** arranged and configured to affect the actuator **120** (by moving or shifting the actuator **120** with respect to the jaws **110** and/or the cam mechanism **150**) to effect shifting of the jaws **110** between closed and open configuration. Longitudinal shifting, along the longitudinal axis **LA** of the tissue-engagement member **100** as well as generally longitudinally along a deployment system **180** (as in **FIG. 1**), is considered one of the simpler motions to effect within a body, such as transluminally.

In the embodiment of a tissue-engagement member **100** illustrated in **FIGS. 2-4****,** the actuation element **122** is coupled to the actuator **120** via a shaft **124.** The shaft **124** extends proximally toward the proximal end **103** of the tissue-engagement member **100** from the actuator **120** through an axial opening **147** at the proximal end **143** of the housing **140** to be coupled with the actuation element **122.** The coupling of the actuation element **122** may be accomplished in any known or heretofore manner such as brazing or welding (e.g., of metal materials) mechanical deformation and coupling (such as crimping) or co-molding (of polymeric materials) or other coupling. In some embodiments, at least the actuation element **122** and the shaft **124** are formed as a unitary, single piece (e.g., the actuation element **122** is bent from or co-molded with the element forming the shaft **124**)**.** In other embodiments, such as described below, an actuation element and shaft are formed as separate elements coupled together in any desired manner known or heretofore known in the art. Proximal retraction of the actuation element **122** (in a direction towards proximal end **103** and away from distal end **101** of the tissue-engagement member **100,** such as may be appreciated upon comparison of movement from the position illustrated in **FIG. 2** to the position illustrated in **FIG. 3**) causes proximal movement of shaft **124** and consequent proximal movement of the actuator **120.** As the actuator **120** moves proximally, the cam followers **154** extending therefrom ride along the cam surfaces **152** provided on the shanks **114** of the jaws **110,** such as from a position as illustrated in **FIG. 2** to a position as illustrated in **FIG. 3****.** As the cam followers **154** ride along the cam surfaces **152,** the jaws **110** are moved against the biasing force of the biasing element **130** from a closed position (as illustrated in **FIG. 2****)** to an open position (such as illustrated in **FIG. 3****).**

The biasing element **130** may be held in place with respect to the respective shanks **114** of the jaw members **110a, 110b** and may exert a closing force on the jaw members **110a, 110b** to return the jaws **110** to a closed configuration. In the example of an embodiment of a tissue-engagement member **100** illustrated in **FIGS. 2** and **3****,** a groove and/or shoulders **118** are provided on at least one of the shanks **114** of the jaw members **110a, 110b** to hold the biasing element **130** in place with respect to the shanks **114** and the cam mechanism **150.** However, other configurations are in the scope of the present disclosure. When a proximal force is no longer applied to the actuation element **122** (e.g., the actuation element **122** is released), the biasing force of the biasing element **130** biases the jaws **110** to return to the closed configuration, and the cam follower **154** rides along the cam surface **152** to move the actuator **120** proximally (e.g., from a position as in **FIG. 3** to a position as in **FIG. 2**)**.**

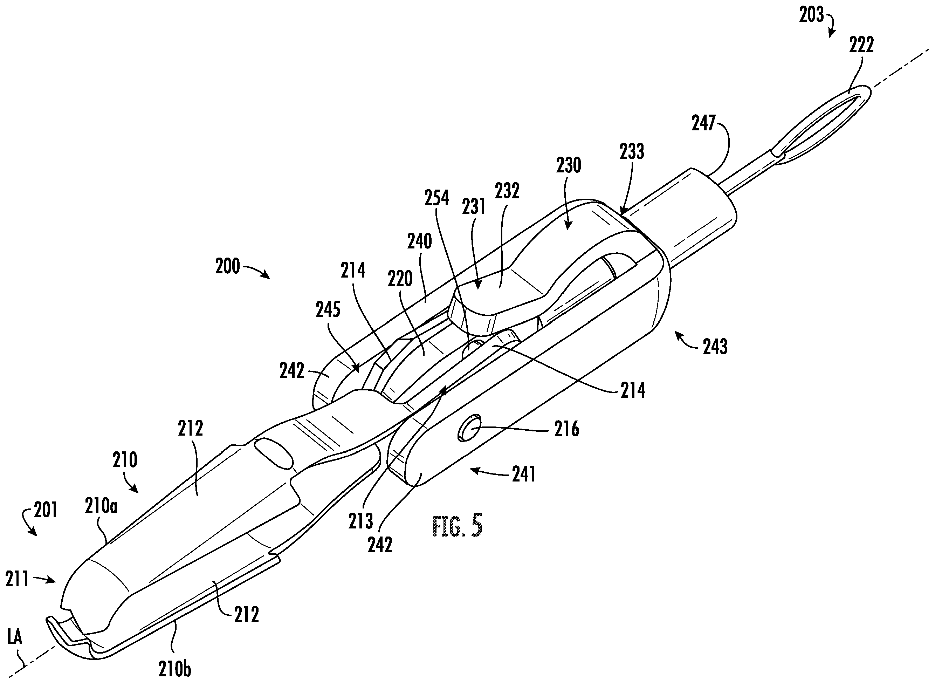

Although the example of an embodiment of a tissue-engagement member **100** illustrated in **FIGS. 2-4** has a biasing element **130** which is separately formed from the cam mechanism **150** and the jaws **110** and the actuator **120** of the tissue-engagement member **100,** and is provided around or about the exterior of the jaws **110,** other configurations and positions and locations of a biasing element used in conjunction with a cam mechanism and/or jaws and/or an actuator of a tissue-engagement member are within the scope of the present disclosure. Moreover, other mechanisms for coupling jaws of a tissue-engagement member with an actuator to effect movement of the jaws between open and closed configurations are within the scope of the present disclosure. Various alternative embodiments are illustrated in **FIGS. 5-18** as described in further detail below.

An alternative example of an embodiment of a tissue-engagement member **200** is illustrated in **FIGS. 5-7** with different configurations of a cam mechanism **250** and a biasing element **230** than the configurations of a cam mechanism **150** and biasing element **130** illustrated in the tissue-engagement member **100** of **FIGS. 2-4****.** It will be appreciated that for the sake of brevity and convenience, and without intent to limit, common elements with common functions in the tissue-engagement member **200** illustrated in **FIGS. 5-7** as in the tissue-engagement member **100** illustrated in **FIGS. 2-4** are indicated with the same reference characters differing in value by 100, reference being made to the above descriptions of similar elements and operations.

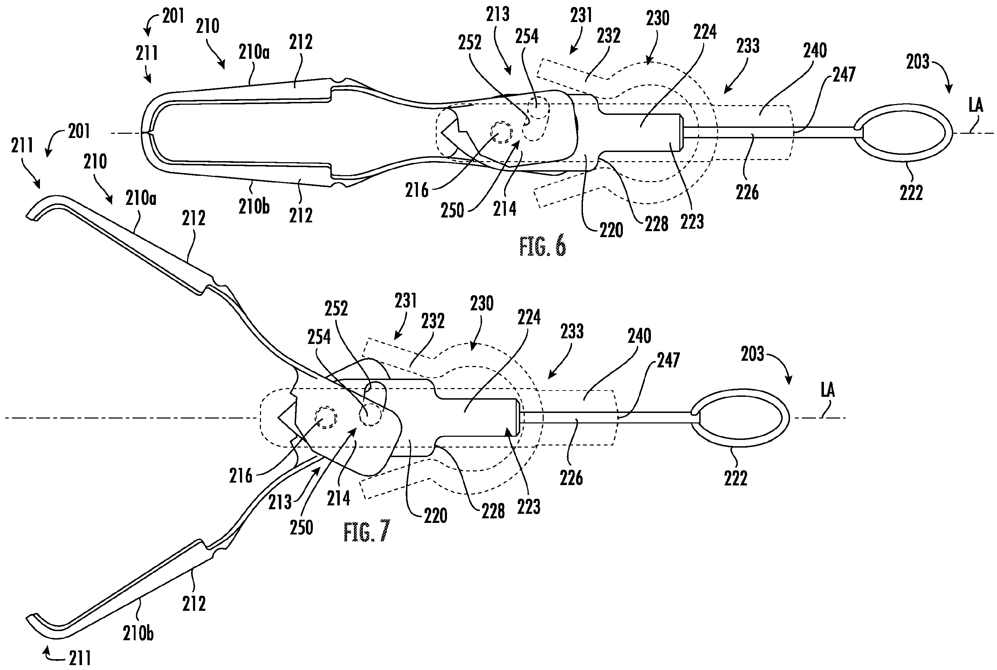

In general, the cam mechanism **250** of the tissue-engagement member **200** of **FIGS. 3-5** may be considered similar to the cam mechanism **150** of the tissue-engagement member **200** of **FIGS. 3-5** in that both cam mechanisms include a cam surface and a cam follower. However, the positions of such cam mechanism elements with respect to the jaws and actuator in the tissue-engagement member **200** are reversed from the positions of such elements in the tissue-engagement member **100.** As may be appreciated with reference to **FIG. 7****,** the cam surface **252** of the cam mechanism **250** is in the form of a slot formed in the actuator **220.** The cam follower **254** is in the form of a pin extending inwardly towards the actuator **220** from the shanks **214** of the jaws **210,** as may be seen partially in **FIG. 5****,** and in phantom in **FIG. 6** and **FIG. 7****.**

As in the embodiment of a tissue-engagement member **100** illustrated in **FIGS. 2-4****,** a biasing element **230** is used in conjunction with the cam mechanism **250** of the example of a tissue-engagement member **200** illustrated in **FIGS. 5-7** to facilitate opening and closing of the jaws **210.** However, in contrast, the biasing element **230** operatively engages the actuator **220,** instead of being operatively engaged with the jaws **210** (as is the biasing element **130** of the tissue-engagement member **100** illustrated in **FIGS. 2**-**4**). Moreover, the biasing element **230** may be operatively engaged with (e.g., mounted on or formed as a part of) the housing **240** to extend from the housing **240** towards the cam mechanism **250.** In the example of an embodiment illustrated in **FIGS. 5-7****,** the biasing element **230** extends from a proximal end **243** of the housing **240** distally towards the distal end **201** of the tissue-engagement member **200.** The proximal end **233** of the biasing element **230** may be coupled to or formed integrally with the proximal end **243** of the housing **240.** A spring arm **232** may be formed along the distal end **231** of the biasing element **230** to interact with the actuator **220,** as will be described in further detail below. As such, it will be appreciated that the housing **240** of the tissue-engagement member **100** illustrated in **FIGS. 3-5** differs somewhat from the housing **140** of the tissue-engagement member **100** illustrated in **FIGS. 2-4****.**

Other than the above-described differences, the other elements of the example of an embodiment of a tissue-engagement member **200** illustrated in **FIGS. 5-7** are similar to corresponding elements of the example of an embodiment of a tissue-engagement member **100** illustrated in **FIGS. 2-4****.** Moreover, operations of these tissue-engagement members are similar in that actuation of the tissue-engagement members to shift between closed and open configurations may be achieved by pulling, generally longitudinally, on an actuation element which is readily graspable by a medical instrument. In particular, a medical instrument (such as illustrated in **FIG. 1** and **FIGS. 19****-21,** as described in further detail below) may be used to move the actuation element **222** longitudinally along the longitudinal axis **LA** in a proximal direction (in a direction towards the proximal end **203** and away from the distal end **201** of the tissue-engagement member **200**). The actuation element **222** is coupled to the actuator **220** via a shaft **224.** Optionally, a shaft extension **226** (e.g., in the form of a wire **226**) may couple the actuation element **222** with the shaft **224** (in any known or heretofore known manner, such as by brazing, welding, crimping, co-molding, passing of shaft extension **226** through a passage within shaft **224,** etc.). In some embodiments, the actuation element **222** and the shaft extension **226** are formed from a common element (e.g., a wire with a proximal loop forming the actuation element **222**). The shaft **224** (and/or the shaft extension **226** if provided) extends proximally through an axial opening **247** at the proximal end **243** of the housing **240** to be coupled with the actuation element **222.** Proximal shifting of the actuation element **222** and shaft **224** cause proximal movement of the actuator **220.** As the actuator **220** moves proximally, the cam followers **254** (on the shanks **214** along the proximal ends **213** of the jaws **210**) ride along the cam surface **252** (formed in the actuator **220**) to cause the jaw members **210a, 210b** to pivot with respect to each other about the pivot pin **216** to shift the tissue-engaging ends **212** to move apart from a closed configuration **(****FIG. 6****)** to an open configuration **(****FIG. 7****).** Also, as the actuator **220** moves proximally to actuate the jaws **210,** the actuator **220** moves proximally with respect to the biasing element **230.** A shoulder **228** on the actuator **220,** facing towards the proximal end **223** of the actuator **220,** may engage with the biasing element **230** to shift the biasing element **230** to a biasing configuration in which the spring arm **232** is biased to an open configuration, storing potential energy, such as illustrated in **FIG. 7****.** Thus, upon release of the actuation element **222,** the potential energy stored in the biasing element **230** causes the spring arm **232** to release the stored potential energy, and to shift the actuator **220** distally to return the tissue-engaging ends **212** to a closed configuration (e.g., with the distal end **211** of the jaws **110** positioned to engage or hold tissue therebetween, such as illustrated in **FIG. 1** or **FIG. 6**).

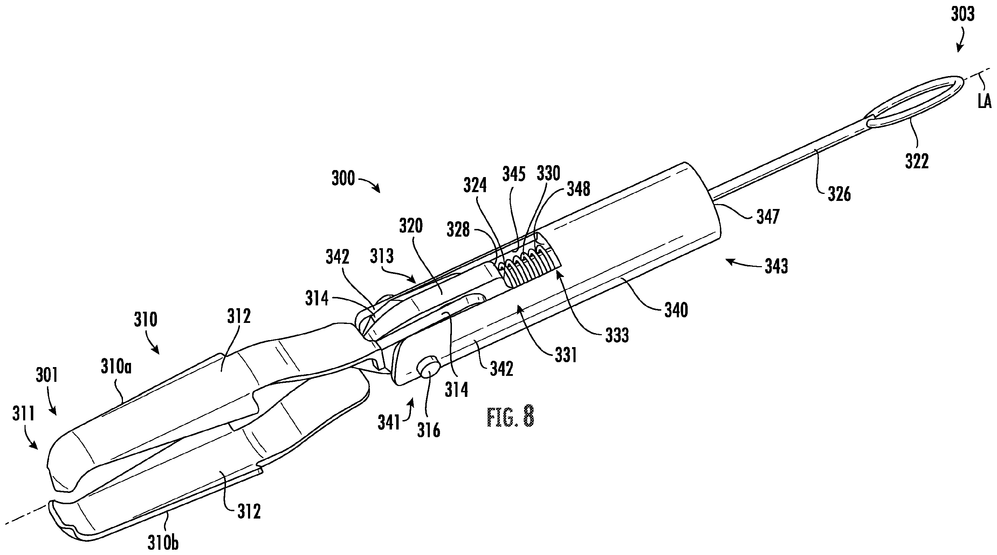

In another example of a tissue-engagement member **300,** illustrated in **FIGS. 8-10****,** a cam mechanism **350** similar to the cam mechanism **250** of the tissue-engagement member **200** illustrated in **FIGS. 5-7****,** may be provided with a variation to the biasing element **330** interacting therewith. Although the biasing element **330** of the tissue-engagement member **300** illustrated in **FIGS. 5-7** is operatively engaged with the actuator **320** as well as the housing **340** (similar to the biasing element **230** of the tissue-engagement member **200** being operatively engaged with the actuator **220** and the housing **240**), the biasing element **330** is formed separately from the housing **340.** More particularly, the biasing element **330** is positioned between the housing **340** and the actuator **320,** such as in a window **345** in the housing **340.** In the illustrated embodiment, the biasing element **330** is positioned about a shaft **324** extending proximally (towards the proximal end **303** of the tissue-engagement member **300**) from the actuator **320,** and may be in the form of a coil spring.

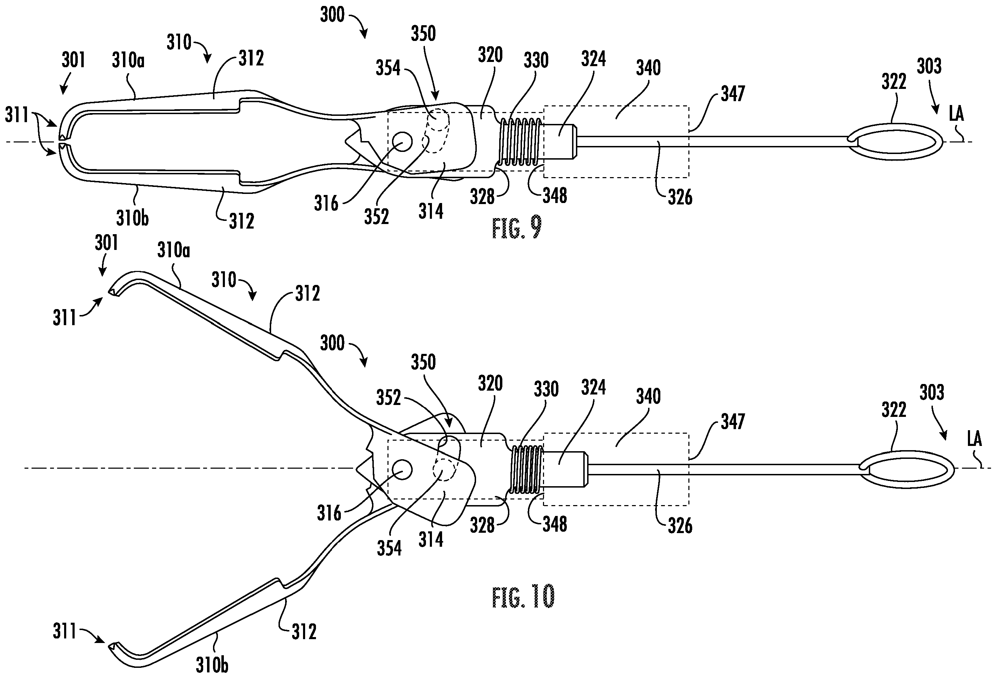

The example of an embodiment of a tissue-engagement member **300** illustrated in **FIGS. 8-10** is actuated to shift the jaws **310** thereof between closed and open configurations in a manner similar to that in which the tissue-engagement member **200** illustrated in **FIGS. 5-7** is actuated. The actuation element **322** may be shifted along the longitudinal axis **LA** of the tissue-engagement member **300** in a proximal direction (in a direction towards the proximal end **303** and away from the distal end **301** of the tissue-engagement member 300). The actuation element **322** is coupled to the actuator **320** via a shaft **324** extending proximally from the actuator **320.** Optionally, a shaft extension **326** (e.g., in the form of a wire **326,** as in the embodiment of **FIGS. 5-7**) extends through an axial opening **347** at the proximal end **343** of the housing **340** to couple the shaft **324** and the actuation element **322.** Proximal shifting of the actuation element **322** and shaft **324** (and optional shaft extension **326**) causes proximal movement of the actuator **320.** As the actuator **320** moves proximally, the cam followers **354** (on the shanks **314** along the proximal ends **313** of the jaws **310**) ride along the cam surface **352** (formed in the actuator **320)** to cause the jaw members **310a, 310b** to pivot with respect to each other about the pivot pin **316** to shift the tissue-engaging ends **312** to move apart from a closed configuration **(****FIG. 8** and **FIG. 9****)** to an open configuration **(****FIG. 10****).** Also, as the actuator **320** moves proximally, the actuator **320** compresses the biasing element **330,** storing potential energy therein, such as illustrated in **FIG. 10****.** More particularly, as illustrated in **FIG. 8****,** the distal end **331** of the biasing element **330** abuts against a shoulder **328** along the actuator **320** facing toward the proximal end **303** of the tissue-engagement member **300,** and a proximal end **333** of the biasing element **330** abuts against a shoulder **348** on the housing **340** facing toward the distal end **301** of the tissue-engagement member **300** as the actuator **320** is shifted proximally. Upon release of the actuation element **322,** the potential energy stored in the biasing element **330** causes the biasing element **330** to return to its uncompressed configuration (as in **FIG. 9**), shifting the actuator **320** distally to return the tissue-engaging ends **312** to a closed configuration (e.g., with the distal ends **311** of the jaws **310** positioned to engage or hold tissue therebetween, such as illustrated in **FIG. 1** or **FIG. 9**)**.**

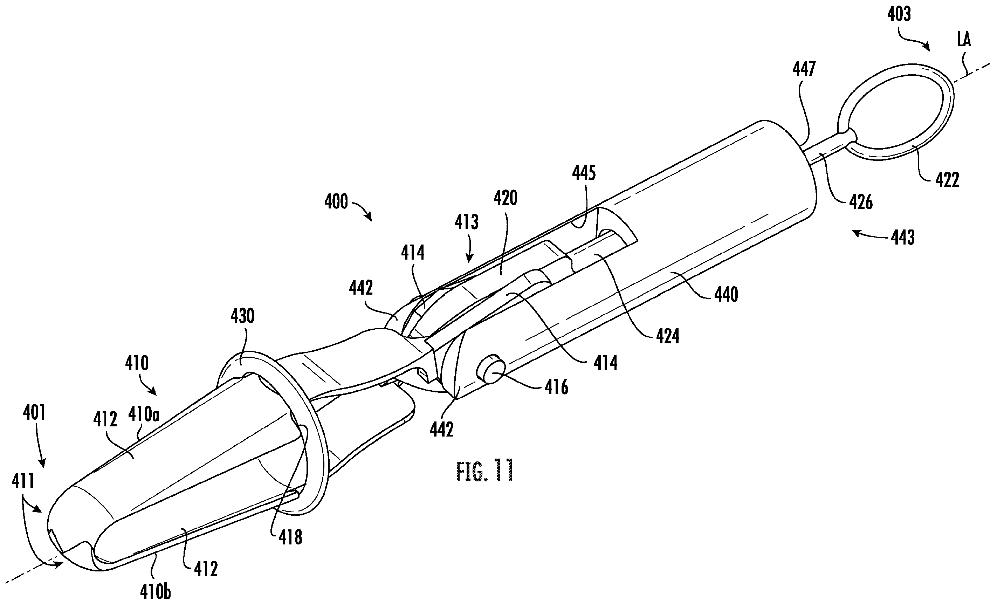

Yet another modification to a biasing element **430** which may be used with a tissue-engagement member having elements similar to the tissue-engagement members **200, 300** of **FIGS. 5-7** and **FIGS. 8-10****,** respectively, is illustrated in the example of a tissue-engagement member **400** of **FIGS. 11-13****.** Instead of being operatively engaged with an actuator **420** (as in the tissue-engagement members **200, 300** of **FIGS. 5-7** and **FIGS. 8-10****),** the biasing element **430** of the tissue-engagement member **400** illustrated in **FIGS. 11-13** is operatively engaged with the jaws **410** of the tissue-engagement member **400.** The illustrated biasing element **430** may be similar to the biasing element **130** of the tissue-engagement member **100** illustrated in **FIGS. 2-4****,** with other elements being similar to the elements of the tissue-engagement members **200, 300** of **FIGS. 5-7** and **FIGS. 8-10****,** respectively. For the sake of brevity and convenience, and without intent to limit, common elements with common functions in the tissue-engagement member **400** illustrated in **FIGS. 11-13** as in the tissue-engagement members **200, 300** illustrated in **FIGS. 5-7** and **FIGS. 8-10****,** respectively, are indicated with the same reference characters differing in value by 100, reference being made to the above descriptions of similar elements and operations.

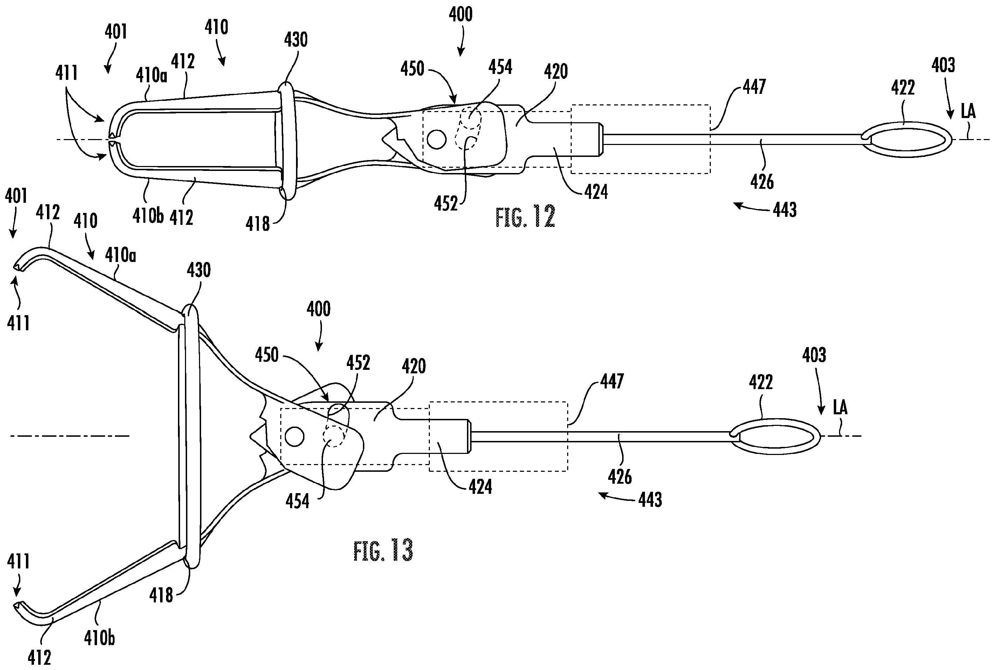

As noted above, the biasing element **430** of the tissue-engagement member **400** illustrated in **FIGS. 11-13** is operatively engaged with the jaws **410** of the tissue-engagement member **400,** like the biasing element **130** of the tissue-engagement member **100** illustrated in **FIGS. 2-4****,** to hold the jaws **410** in a closed configuration. However, instead of being operatively engaged with jaw shanks and/or a housing (as in the tissue-engagement member **100** of **FIGS. 2-****4),** the biasing element **430** is operatively engaged with the tissue-engaging ends **412** of the jaws **410.** More particularly, the biasing element **430** may be positioned about the tissue-engaging ends **412** of the jaws **410** (e.g., surrounding the jaws **410**) to exert a biasing force to hold the jaws **410** in a closed configuration as illustrated in **FIG. 11** and **FIG. 12****.** In the example of an embodiment of a tissue-engagement member **400** illustrated in **FIGS. 11-13****,** a groove and/or shoulders **418** are provided on at least one of the tissue-engaging ends **412** of the jaw members **410a, 410b** to hold the biasing element **430** in place with respect to the jaws **410.** However, other configurations are in the scope of the present disclosure.

Actuation of the example of an embodiment of a tissue-engagement member **400** illustrated in **FIGS. 11-13** is similar to that of the tissue-engagement members **200, 300** illustrated in **FIGS. 5-7** and **8-10,** other than the location of the biasing force returning the jaws **410** to a closed configuration. The actuation element **422** along the proximal end **403** of the tissue-engagement member **400** may be shifted along the longitudinal axis **LA** of the tissue-engagement member **400** in a proximal direction (in a direction towards the proximal end **403** and away from the distal end **401** of the tissue-engagement member **400**) to shift an actuator **420** operatively associated with the jaws **410** to shift the jaws **410** between a closed configuration (as in **FIGS. 11** and **12****)** and an open configuration (as in **FIG. 13****).** The actuation element **422** is coupled to the actuator **420** via a shaft **424** extending proximally from the actuator **420.** Optionally a shaft extension **426** (e.g., in the form of a wire **426,** as in the embodiment of **FIGS. 5-7****)** extends proximally through an axial opening **447** at the proximal end **443** of the housing **440** to couple the shaft **424** and the actuation element **422.** Proximal shifting of the actuation element **422** causes proximal shifting of the actuator **420.** As the actuator **420** moves proximally, the cam followers **454** (on the shanks **414** along the proximal ends **413** of the jaws **410**) ride along the cam surface **452** (formed in the actuator **420)** to cause the jaw members **410a, 410b** to pivot with respect to each other about the pivot pin **416** to shift the tissue-engaging ends **412** to move apart from a closed configuration **(****FIG. 11** and **FIG. 12****)** to an open configuration **(****FIG. 13****).** As the jaws **410** open, the biasing element **430** is stretched, storing potential energy therein, such as illustrated in **FIG. 13****.** Upon release of the actuation element **422,** the potential energy stored in the biasing element **430** causes the biasing element **430** to return to its uncompressed configuration (as in **FIG. 12**), moving the tissue-engaging ends **412** to a closed configuration (e.g., with the distal ends **411** of the jaws **410** positioned to engage or hold tissue therebetween, such as illustrated in **FIG. 1** or **FIG. 11** or **FIG. 12****).**

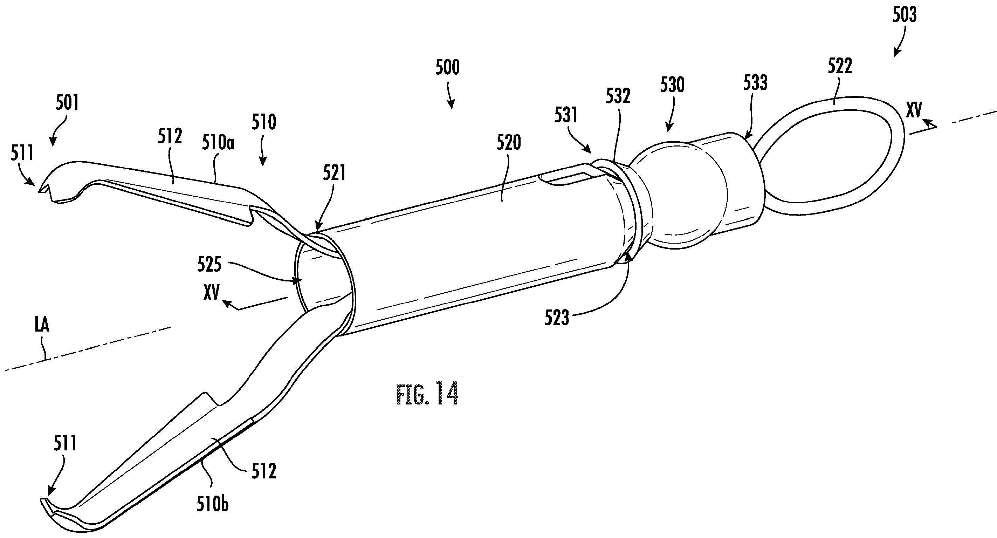

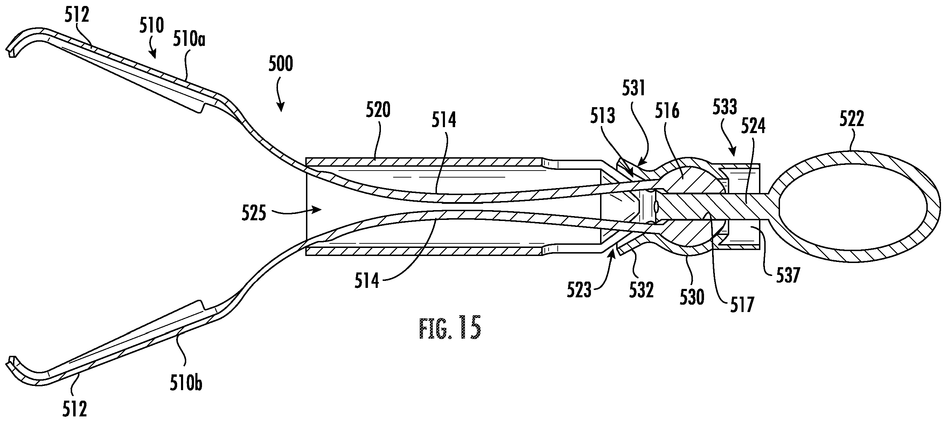

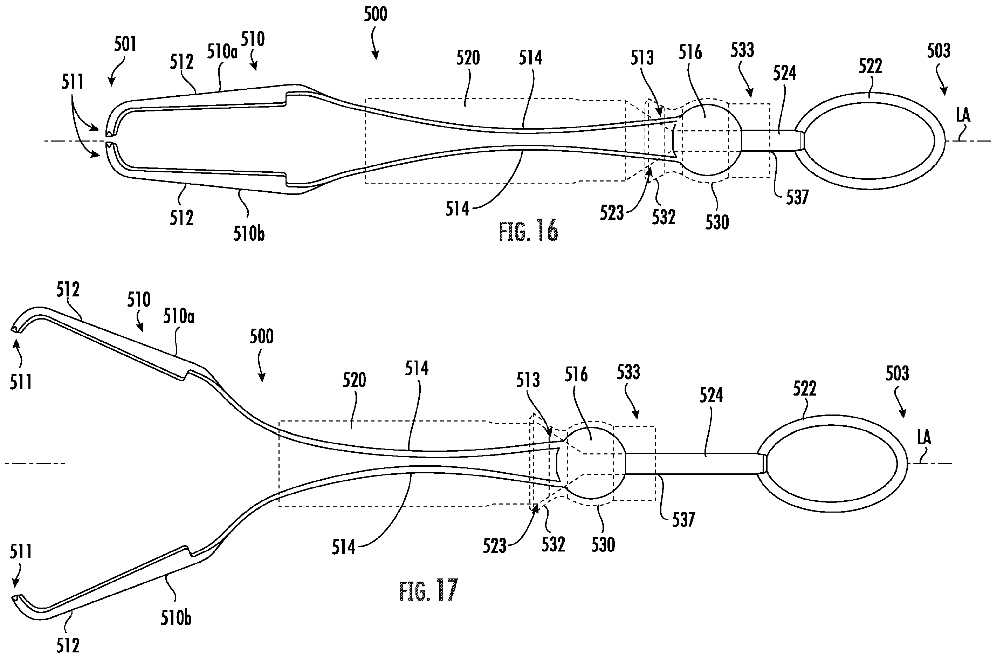

In another embodiment of a tissue-engagement member **500** formed in accordance with various principles of the present disclosure, and as illustrated in **FIGS. 14-18****,** the jaws **510** may be configured or formed to be naturally biased into an open configuration (as in **FIGS. 14****,** **15****,** **17****,** and **18****).** The actuator **520** may be in the form of a retention member or fillet, shiftable to retain the jaws **510** in a closed configuration (as in **FIG. 16****)** or to allow the jaws **510** to expand into the open configuration (as in **FIGS. 14****,** **15****,** **17****,** and **18**)**.** The biasing element **530** may be in the form of a spring-biased collet or boss or solid spring (e.g., conical solid spring) with a spring opening **532** operatively engaging the actuator **120** to bias the actuator **520** to return to a configuration closing the jaws **510.** Details of the example of an embodiment of a tissue-engagement member **500** illustrated in **FIG. 14** may be appreciated with reference to the cross-sectional view illustrated in **FIG. 15** along line **XV-XV** of **FIG. 14****,** and the elevational views illustrated in **FIG. 16** and **FIG. 17** with the actuator **520** in phantom.

In the embodiment illustrated in **FIG. 15****,** the jaws **510** of the illustrated tissue-engagement member **500** have jaw members **510a, 510b** (between which tissue may be grasped when the jaws **510** are in a closed configuration) adjacent the distal end **511** of the jaws **110** with shanks **514** extending proximally towards the proximal end **513** of the jaws **510** at which the shanks **514** are coupled via a jaw ball joint **516.** The shanks **514** are configured to bias the jaw members **510a, 510b** apart when unconstrained. The actuator **520** is positioned relative to the jaws **510** and relative to the biasing element **530** to retain the jaws **510** in a closed configuration, as illustrated in **FIG. 16****.** To open the jaws **510,** an actuation element **522,** which may be similar to the above-described actuation elements **122, 222, 322, 422** (reference being made to details of such actuation elements described above as being optionally applicable to the actuation element **522**), is shifted along the longitudinal axis **LA** in a proximal direction (in a direction towards the proximal end **503** of the tissue-engagement member **500** and away from the distal end **501** of the tissue-engagement member **500**). The actuation element **522** is coupled to the actuator **520** via a shaft **524** coupled to a proximal end **523** of the actuator **520** (and extending through an opening **537** in the proximal end **533** of the biasing element **530** as well as an opening **517** through the jaw ball joint **516** of the jaws **510).** Proximal shifting of the actuation element **522** and the shaft **524** shift the actuator **520** proximally towards the spring opening **532** along the distal end **531** of the biasing element **530** so that the actuator **520** biases the spring opening **532** into an expanded tensioned configuration, storing potential energy. The interaction between the actuator **520** and the spring opening **532** of the biasing element **530** may be considered a camming action such that the tissue-engagement member **500** may be considered to have a cam mechanism operatively associated with the actuator **520** and the biasing element **530.** Upon release of the actuation element **522,** the potential energy stored in the spring opening **532** of the biasing element **530** causes the spring opening **532** to return to its rest configuration, shifting the actuator **520** distally over the shank **514** of the jaws **510** to return the tissue-engaging ends **512** to a closed configuration (e.g., with the distal ends **511** of the jaws **510** positioned to engage or hold tissue therebetween), such as illustrated in **FIG. 1** or **FIG. 16****.**

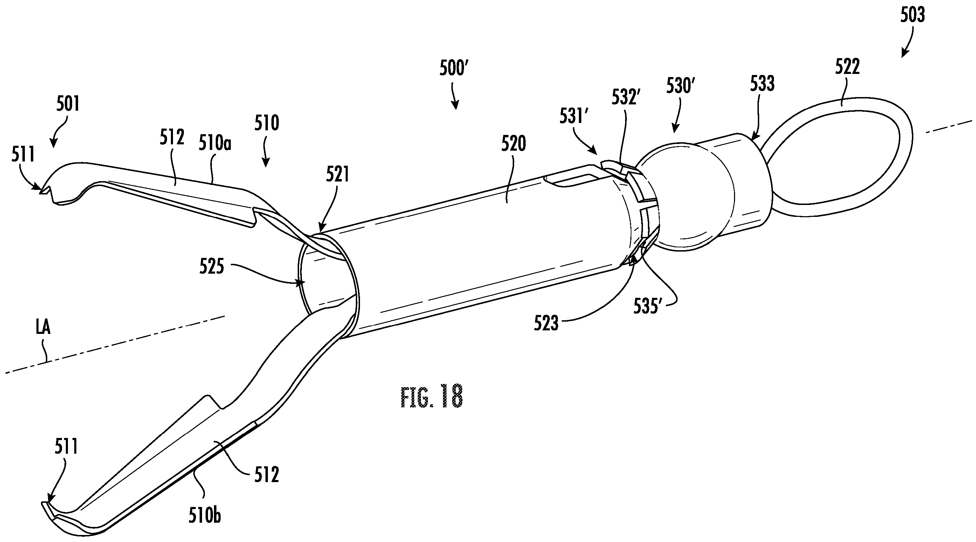

A modified version of a tissue-engagement member **500** as in **FIGS. 14-17** is illustrated in **FIG. 18****.** The modified tissue-engagement member **500'** of **FIG. 18** has a modified biasing element **530'** in which the spring opening **532'** includes cuts or slits **535'** along the distal end **531'** thereof to facilitate expansion of the spring opening **532'** as the actuator **520** extends proximally into the spring opening **532'** upon shifting the actuator **520** and shaft **524** proximally along the longitudinal axis **LA** (toward the proximal end **503** and away from the distal end **501** of the tissue-engagement member **500**). It will be appreciated that the remaining elements of the tissue-engagement member **500'** illustrated **in** **FIG. 18** are substantially the same as similarly labeled elements of the tissue-engagement member **500** illustrated in **FIGS. 14-17****,** reference being made to the above descriptions of such elements as applicable to the embodiment of **FIG. 18****.**

Reference is made to **FIG. 1** and **FIGS. 19****-20** to describe deployment of a tissue-engagement member formed in accordance with various principles of the present disclosure. It will be appreciated, in view of the above, that any of the above-described tissue-engagement members **200, 300, 400, 500, 500'** may be deployed in a similar manner. In the example illustrated **in** **FIG. 1****,** more than one tissue-engagement member **100** may be deployed in a similar manner with a tether **160** extending between the tissue-engagement members **100.** The tether **160** may be a tissue traction element configured to apply traction to tissue **T** engaged by the first-deployed of the tissue-engagement members **100** (on the left in **FIG. 1**) upon engaging the second-deployed of the tissue-engagement members **100** (on the right in **FIG. 2**) with tissue spaced apart from the first-engaged tissue-engagement member **100.** It will be appreciated that other uses and environments for a tissue-engagement member formed in accordance with various principles of the present disclosure are within the scope of the present disclosure.

In accordance with an aspect of the present disclosure, a tissue engagement system includes one or more tissue-engagement members **100** and a tissue-engagement-member manipulator **170** configured to actuate at least one of the tissue-engagement members **100,** as illustrated in **FIG. 1****.** One or more tissue-engagement members **100** may be deployed to a deployment site, optionally along with the manipulator **170** (e.g., in the same delivery device). Optionally, the manipulator **170** is deployed with a manipulator deployment system **180** configured to deliver as well as to maneuver the manipulator **170** to actuate the tissue-engagement member **100.** It will be appreciated that the term maneuver and conjugations thereof is used for the sake of convenience without intent to limit, and may be used interchangeably herein with terms such as manipulate, move, control, actuate, steer, navigate, etc., including various conjugations thereof. For the sake of simplicity, the deployment system with which the tissue-engagement members **100** and/or manipulator **170** and the deployment system **180** are deployed is not shown. It will be appreciated that any known or heretofore known deployment system may be used, such as any of a variety of flexible tubular elements, typically with optical / visualization capabilities (e.g., an endoscope or other introducer), the details of such system not being critical to the principles of the present disclosure. The tissue-engagement members **100** and manipulator **170** and deployment system **180** may be navigated to / deployed at a deployment site via separate systems (e.g., separate delivery systems) or separate working channels in a common system. The present disclosure is not limited in this context.

As illustrated in **FIG. 1****,** the manipulator **170** is extended out the distal end **181** of the manipulator deployment system **180** to engage the actuation element **122** of a tissue-engagement member **100.** An actuator-engaging element **172** is provided along a distal end **171** of the manipulator **170** to operatively engage an actuation element **122** so that proximal shifting of the manipulator **170** pulls the actuation element **122** proximally to actuate the tissue-engagement member **100** to open in a manner as described above. In accordance with an aspect of the present disclosure, the actuator-engaging element **172** is configured to operatively engage the actuation element **122** of the tissue-engagement member **100** within a range of angular orientations with respect to the actuation element **122.** In other words, the configurations of the actuation element **122** and the actuator-engaging element **172** allow for a range of relative positions with respect to each other which still allow for operative engagement therebetween. In some embodiments, the actuator-engaging element **172** is in the form of a hook (such as formed along a distal end of a pull wire) which is engaged with the actuation element **122,** such as by being inserted into an opening **125** within the actuation element **122.** As may be appreciated by those of ordinary skill in the art, the hook (generally, along a major plane in which a majority of the hook lies) merely needs to be transverse (i.e., not parallel) to the actuation element **122** (generally, the major plane in which the actuation element **122** lies, or at least the portion of the actuation element **122** to be grasped by the actuator-engaging element **172** of the manipulator **170**). As such, a range of relative orientations of the actuator-engaging element **172** and the actuation element **122** allow operative engagement therebetween to allow movement of the manipulator **170** to effect movement of the actuation element **122.** More particularly, the actuator-engaging element **172** may be up to just a few degrees short of 90° (as may generally be determined by the thickness of the actuator-engaging element **172,** such as about 5°), or just a few degrees short of 270° to allow the actuator-engaging element **172** to grab and remain engaged with the actuation element **122** (as may generally be determined by the thickness of the actuator-engaging element **172,** such as about 5°), from an ideally aligned position with the hook substantially perpendicular to the actuation element **122** with the open side of the hook oriented to hook over a portion of the actuation element **122.**

In the example of an embodiment of a deployment system **180** illustrated in **FIG. 1****,** a flexible elongate element **174** of the manipulator **170** extends proximally along a flexible tubular element **182,** such as through a lumen **185** (partially shown, in phantom) within the flexible tubular element **182,** to be controlled at a proximal end **183** of the deployment system **180** by a medical professional, such as via a control handle **190.** The flexible tubular element **182** may be a catheter or other element with a lumen **185** therein, preferably capable of being navigated through a body passage or body lumen (e.g., through the gastrointestinal system), such as known or heretofore known in the art. The flexible tubular element **182** may include a coil section (with the lumen **185** extending therethrough) to facilitate transluminal navigation through tortuous pathways, such as known in the art. A sheath **184** may extend over the length of the flexible tubular element **182.**

In the example of an embodiment of a control handle **190** illustrated in **FIG. 1****,** a rotating knob **192** coupled with the flexible elongate element **174** of the manipulator **170** may be used to rotate the actuator-engaging element **172** of the manipulator **170** for positioning with respect to the actuation element **122** to operatively engage the actuation element **122.**

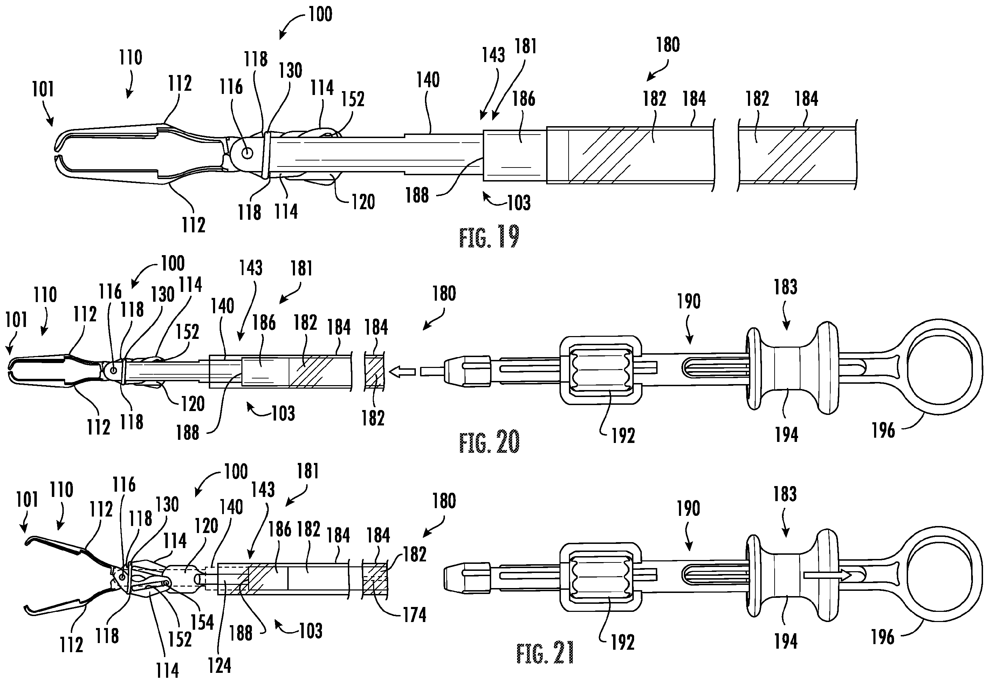

Once the actuator-engaging element **172** and the actuation element **122** are operatively engaged (such as illustrated in **FIG. 1**), the manipulator **170** may be retracted proximally to retract the actuator **120** of the tissue-engagement member **100** proximally. For instance, the control handle **190** may include a translating knob **194** axially translatable along the control handle **190** (in a direction between the proximal end **183** and the distal end **181** of the deployment system **180**) to move the manipulator **170** distally or proximally. In the example illustrated in **FIG. 19** (showing the tissue engagement system enlarged relative to the depictions of **FIG. 20** and **FIG. 21****,** but without illustrating the control handle **190**), the tissue-engagement member **100** is proximally retracted by the manipulator **170** to engage the proximal end **143** of the housing **140** of the tissue-engagement member **100** with a distal section **186** at a distal end **181** of the flexible tubular element **182.** In some embodiments, the flexible tubular element **182** is flexible, such as formed from a coil element (such as described above), and the distal section **186** is less flexible than the portions of the flexible tubular element **182** proximal thereto. For instance, the distal section **186** may be tubular element, such as a bushing, without the springy resiliency and potential axial mobility of the portions of the flexible tubular element **182** extending proximally therefrom. The manipulator **170** may be retracted into the lumen **185** of the flexible tubular element **182** and the distal section **186,** but the tissue-engagement member **100** does not fit within the lumen **185,** as may be appreciated with reference to **FIG. 19****.** Instead, the distal end of the distal section **186** provides a shoulder **188** against which the proximal end **143** of the housing **140** of the tissue-engagement member **100** abuts when moved proximally toward the deployment system **180.** Such arrangement allows for relative longitudinal movement of the manipulator **170** with respect to the housing **140** of the tissue-engagement member **100.**

An alignment sheath **184** may be distally extended (such as by being axially shifted at a proximal end thereof manually or via an associated controller, such as a control handle or control knob) over the housing **140** of the tissue-engagement member **100** to hold the tissue-engagement member **100** in place relative to the deployment system **180,** as illustrated in **FIG. 20****.** With the housing **140** held against relative movement with respect to the flexible tubular element **182,** proximal retraction of the manipulator **170** relative to the flexible tubular element **182** allows the manipulator **170** to pull the actuator **120** relative to the housing **140** of the tissue-engagement member **100.** The translating knob **194** may be moved proximally with respect to the flexible tubular element **182** to effect proximal movement of the flexible elongate element **174** (shown in phantom extending through the flexible tubular element **182** and sheath **184**)**.** The actuation element **122** and the shaft **124** (extending proximally from the actuator **120**) may fit within the lumen **185** of the flexible tubular element **182** to allow the actuator **120** to move proximally relative to the housing **140.** In a manner as described above, proximal shifting of the actuator **120** relative to the jaws **110** (which are pivotably coupled to the housing **140** and thus held with the housing **140** against proximal movement) causes the jaws **110** to shift to an open configuration such as illustrated in **FIG. 21****.** Reference is made above to further details of shifting of the actuator **120** causing the cam followers **154** on the actuator **120** to ride along the cam surfaces **152** on the respective shanks **114** of the jaws **110** to shift the jaws **110** to an open configuration, relevant reference numerals being provided in **FIGS. 19-21** for referencing with respect to the above descriptions of actuation of the jaws **110.**

It will be appreciated that various structures and features of the embodiments described herein and illustrated in the figures have several separate and independent unique benefits. Therefore, the various structures and features described herein need not all be present in order to achieve at least some of the desired characteristics and/or benefits described herein. Moreover, the various features described herein may be used singly or in any combination. It will be appreciated that various features described with respect to one embodiment may be applied to another embodiment, whether or not explicitly indicated. Thus, it should be understood that one or more of the features described with reference to one embodiment can be combined with one or more of the features of any of the other embodiments described herein. That is, any of the features described herein can be mixed and matched to create hybrid designs, and such hybrid designs are within the scope of the present disclosure. Therefore, the present invention is not limited to only the embodiments specifically described herein. The above descriptions are of illustrative examples of embodiments only, and are not intended as limiting the broader aspects of the present disclosure.

The foregoing discussion has broad application and has been presented for purposes of illustration and description and is not intended to limit the disclosure to the form or forms disclosed herein. It will be understood that various additions, modifications, and substitutions may be made to embodiments disclosed herein without departing from the concept and scope of the present disclosure. In particular, it will be clear to those skilled in the art that principles of the present disclosure may be embodied in other forms, structures, arrangements, proportions, and with other elements, materials, and components, without departing from the concept, scope, or characteristics thereof. For example, various features of the disclosure are grouped together in one or more aspects, embodiments, or configurations for the purpose of streamlining the disclosure. However, it should be understood that various features of the certain aspects, embodiments, or configurations of the disclosure may be combined in alternate aspects, embodiments, or configurations. While the disclosure is presented in terms of embodiments, it should be appreciated that the various separate features of the present subject matter need not all be present in order to achieve at least some of the desired characteristics and / or benefits of the present subject matter or such individual features. One skilled in the art will appreciate that the disclosure may be used with many modifications or modifications of structure, arrangement, proportions, materials, components, and otherwise, used in the practice of the disclosure, which are particularly adapted to specific environments and operative requirements without departing from the principles or scope of the present disclosure. For example, elements shown as integrally formed may be constructed of multiple parts or elements shown as multiple parts may be integrally formed, the operation of elements may be reversed or otherwise varied, the size or dimensions of the elements may be varied. Similarly, while operations or actions or procedures are described in a particular order, this should not be understood as requiring such particular order, or that all operations or actions or procedures are to be performed, to achieve desirable results. Additionally, other implementations are within the scope of the following claims. In some cases, the actions recited in the claims can be performed in a different order and still achieve desirable results. The presently disclosed embodiments are therefore to be considered in all respects as illustrative and not restrictive, the scope of the claimed subject matter being indicated by the appended claims, and not limited to the foregoing description or particular embodiments or arrangements described or illustrated herein. In view of the foregoing, individual features of any embodiment may be used and can be claimed separately or in combination with features of that embodiment or any other embodiment, the scope of the subject matter being indicated by the appended claims, and not limited to the foregoing description.

In the foregoing description and the following claims, the following will be appreciated. The phrases "at least one", "one or more", and "and/or", as used herein, are open-ended expressions that are both conjunctive and disjunctive in operation. The terms "a", "an", "the", "first", "second", etc., do not preclude a plurality. For example, the term "a" or "an" entity, as used herein, refers to one or more of that entity. As such, the terms "a" (or "an"), "one or more" and "at least one" can be used interchangeably herein. All directional references (e.g., proximal, distal, upper, lower, upward, downward, left, right, lateral, longitudinal, front, back, top, bottom, above, below, vertical, horizontal, radial, axial, clockwise, counterclockwise, and/or the like) are only used for identification purposes to aid the reader's understanding of the present disclosure, and/or serve to distinguish regions of the associated elements from one another, and do not limit the associated element, particularly as to the position, orientation, or use of this disclosure. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. Identification references (e.g., primary, secondary, first, second, third, fourth, etc.) are not intended to connote importance or priority, but are used to distinguish one feature from another.

The following claims are hereby incorporated into this Detailed Description by this reference, with each claim standing on its own as a separate embodiment of the present disclosure. In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous. In addition, singular references do not exclude a plurality. Reference signs in the claims are provided merely as a clarifying example and shall not be construed as limiting the scope of the claims in any way.

## Claims

1. A tissue-engagement member (500, 500') comprising:
a pair of jaw members (510a-b) coupled together and movable between a closed configuration and an open configuration, wherein said jaw members (5 10a-b) are configured or formed to be naturally biased apart from each other;
an actuator (520) operatively engaged with said pair of jaw members to shift said jaw members between the closed configuration and the open configuration, the actuator (520) being movable between a position holding said jaw members in the closed configuration and a position allowing said jaw members to move apart to the open configuration; and
**characterised in that** the tissue-engagement member further comprises
a biasing element (530, 530') positioned over a portion of said actuator (520) to cause said jaw members to be biased into the closed configuration, wherein said biasing element (530, 530') biases said actuator into the position holding said jaw members in the closed configuration.

2. The tissue-engagement member (500, 500') of claim 1, wherein said biasing element (530, 530') is in the form of a spring-biased collet or boss or solid spring with a spring opening (532, 532') operatively engaging the actuator (520) to bias the actuator (520) to return to a configuration closing the jaws.

3. The tissue-engagement member (500') of claim 2, wherein said the spring opening (532') includes cuts or slits (535') along the distal end (531') of the biasing element (530').

4. A tissue-engagement system comprising the tissue-engagement member 500, 500') of any one of claims 1-3, and further comprising:
a tissue-engagement-member manipulator (170) having a distal end configured to operatively engage with said tissue-engagement member to move said actuator to shift said jaws between the closed and open configurations; and
a manipulator deployment system (180) configured to deliver said tissue-engagement-member manipulator (170) and to maneuver said tissue-engagement-member manipulator (170) to move said actuator.

5. The tissue-engagement system of claim 4, wherein:
an actuation element (122) is operatively coupled with a proximal end (103) of said actuator along the proximal end of said tissue-engagement member; and
an actuator-engagement element (172) is provided on a distal end (171) of said tissue-engagement-member manipulator and configured to engage with said actuation element in a range of orientations with respect to said actuation element.

6. The tissue-engagement system of any of claims 4-5, wherein said manipulator deployment system (180) further comprises a flexible tubular element (182) with a lumen (185) defined therethrough, and a sheath (184), wherein said tissue-engagement-member manipulator (170) is movable proximally into the lumen defined through said flexible tubular element (182) to bring the proximal end of said tissue-engagement member into abutment with a distal end (181) of said flexible tubular element (182), and said sheath (184) is movable over the distal end (181) of said flexible tubular element and over said tissue-engagement member to hold said tissue-engagement member in alignment with said manipulator deployment system (180).

## Patentansprüche

1. Gewebeeingriffsteil (500, 500'), das aufweist:
ein Paar Backenteile (510a-b), die miteinander gekoppelt und zwischen einer geschlossenen Konfiguration und einer offenen Konfiguration beweglich sind, wobei die Backenteile (510a-b) so konfiguriert oder ausgebildet sind, dass sie von einander weg natürlich vorgespannt sind;
einen Aktuator (520), der einen betriebswirksamen Eingriff mit dem Paar von Backenteilen herstellt, um die Backenteile zwischen der geschlossenen Konfiguration und der offenen Konfiguration zu verschieben, wobei der Aktuator (520) zwischen einer die Backenteile in der geschlossenen Konfiguration haltenden Position und einer Position beweglich ist, die den Backenteilen ermöglicht, sich in die offene Konfiguration auseinander zu bewegen; und
**dadurch gekennzeichnet, dass** das Gewebeeingriffsteil ferner ein Vorspannelement (530, 530') aufweist, das über einem Abschnitt des Aktuators (520) positioniert ist, um zu bewirken, dass die Backenteile in die geschlossene Konfiguration vorgespannt sind, wobei das Vorspannelement (530, 530') den Aktuator in die die Backenteile in der geschlossenen Konfiguration haltende Position vorspannt.

2. Gewebeeingriffsteil (500, 500') nach Anspruch 1, wobei das Vorspannelement (530, 530') die Form einer federvorgespannten Spannzange oder Nabe oder Massivfeder mit einer Federöffnung (532, 532') hat, die einen betriebswirksamen Eingriff mit dem Aktuator (520) herstellt, um den Aktuator (520) vorzuspannen, um in eine die Backen schließende Konfiguration zurückzukehren.

3. Gewebeeingriffsteil (500') nach Anspruch 2, wobei die Federöffnung (532') Schnitte oder Schlitze (535') entlang des distalen Endes (531') des Vorspannelements (530') aufweist.

4. Gewebeeingriffssystem mit dem Gewebeeingriffsteil (500, 500') nach einem der Ansprüche 1 bis 3 und ferner mit:
einem Gewebeeingriffsteil-Manipulator (170) mit einem distalen Ende, das so konfiguriert ist, dass es einen betriebswirksamen Eingriff mit dem Gewebeeingriffsteil herstellt, um den Aktuator zu bewegen, um die Backen zwischen der geschlossenen und offenen Konfiguration zu verschieben; und
ein Manipulator-Setzsystem (180), das konfiguriert ist, den Gewebeeingriffsteil-Manipulator (170) zuzuführen und den Gewebeeingriffsteil-Manipulator (170) manövriert, um den Aktuator zu bewegen.

5. Gewebeeingriffssystem nach Anspruch 4, wobei:
ein Betätigungselement (122) mit einem proximalen Ende (103) des Aktuators entlang des proximalen Endes des Gewebeeingriffsteils betriebswirksam gekoppelt ist;
und
ein Aktuator-Eingriffselement (172) auf einem distalen Ende (171) des Gewebeeingriffsteil-Manipulators vorgesehen und so konfiguriert ist, dass es einen Eingriff mit dem Betätigungselement über einen Bereich von Orientierungen relativ zum Betätigungselement herstellt.

6. Gewebeeingriffssystem nach einem der Ansprüche 4 bis 5, wobei das Manipulator-Setzsystem (180) ferner ein flexibles Schlauchelement (182) mit einem dadurch definierten Lumen (185) und eine Hülle (184) aufweist, wobei der Gewebeeingriffsteil-Manipulator (170) in das durch das flexible Schlauchelement (182) hindurch definierte Lumen proximal beweglich ist, um das proximale Ende des Gewebeeingriffsteils in Anlage mit einem distalen Ende (181) des flexiblen Schlauchelements (182) zu bringen, und die Hülle (184) über dem distalen Ende (181) des flexiblen Schlauchelements und über dem Gewebeeingriffsteil beweglich ist, um das Gewebeeingriffsteil in Ausrichtung zum Manipulator-Setzsystem (180) zu halten.

## Revendications

1. Élément de contact tissulaire (500, 500') comprenant:
une paire d'éléments de mâchoires (510a-b) couplés ensemble et mobiles entre une configuration fermée et une configuration ouverte, dans lequel lesdits éléments de mâchoires (510a-b) sont configurés ou formés pour être naturellement sollicités écartés l'un de l'autre;
un actionneur (520) en contact fonctionnel avec ladite paire d'éléments de mâchoires pour déplacer lesdits éléments de mâchoires entre la configuration fermée et la configuration ouverte, l'actionneur (520) étant mobile entre une position maintenant lesdits éléments de mâchoires dans la configuration fermée et une position permettant auxdits éléments de mâchoires de s'écarter l'un de l'autre vers la configuration ouverte; et **caractérisé en ce que** l'élément de contact tissulaire comprend en outre
un élément de sollicitation (530, 530') positionné sur une partie dudit actionneur (520) pour amener lesdits éléments de mâchoires à être sollicités dans la configuration fermée, dans lequel ledit élément de sollicitation (530, 530') sollicite ledit actionneur dans la position maintenant lesdits éléments de mâchoires dans la configuration fermée.

2. Élément de contact tissulaire (500, 500') selon la revendication 1, dans lequel ledit élément de sollicitation (530, 530') est sous la forme d'une pince sollicitée par ressort ou d'un bossage ou d'un ressort solide avec une ouverture de ressort (532, 532') en contact fonctionnellement avec l'actionneur (520) pour solliciter l'actionneur (520) pour retourner dans une configuration fermant les mâchoires.

3. Élément de contact tissulaire (500') selon la revendication 2, dans lequel ladite ouverture de ressort (532') inclut des découpes ou des fentes (535') le long de l'extrémité distale (531') de l'élément de sollicitation (530').

4. Système de contact tissulaire comprenant l'élément de contact tissulaire (500, 500') selon l'une quelconque des revendications 1 à 3, et comprenant en outre:
un manipulateur d'élément de contact tissulaire (170) ayant une extrémité distale configurée pour coopérer fonctionnellement avec ledit élément de contact tissulaire pour déplacer ledit actionneur pour déplacer lesdites mâchoires entre la configuration fermée et ouverte; et
un système de déploiement de manipulateur (180) configuré pour délivrer ledit manipulateur d'élément de contact tissulaire (170) et pour manœuvrer ledit manipulateur d'élément de contact tissulaire (170) pour déplacer ledit actionneur.

5. Système de contact tissulaire selon la revendication 4, dans lequel:
un élément d'actionnement (122) est couplé fonctionnellement à une extrémité proximale (103) dudit actionneur le long de l'extrémité proximale dudit élément de contact tissulaire; et
un élément de contact d'actionneur (172) est prévu sur une extrémité distale (171) dudit manipulateur d'élément de contact tissulaire et configuré pour venir en contact avec ledit élément d'actionnement dans une gamme d'orientations par rapport audit élément d'actionnement.

6. Système de contact tissulaire selon l'une quelconque des revendications 4 et 5, dans lequel ledit système de déploiement de manipulateur (180) comprend en outre un élément tubulaire flexible (182) avec une lumière (185) définie à travers celui-ci, et une gaine (184), dans lequel ledit manipulateur d'élément de contact tissulaire (170) est mobile de manière proximale dans la lumière définie à travers ledit élément tubulaire flexible (182) pour amener l'extrémité proximale dudit élément de contact tissulaire en butée contre une extrémité distale (181) dudit élément tubulaire flexible (182), et ladite gaine (184) est mobile sur l'extrémité distale (181) dudit élément tubulaire flexible et sur ledit élément de contact tissulaire pour maintenir ledit élément de contact tissulaire en alignement avec ledit système de déploiement de manipulateur (180).
